# EUROPÄISCHE PATENTSCHRIFT

(11) **EP 1 463 542 B1**
(45) Veröffentlichungstag und Bekanntmachung des Hinweises auf die Patenterteilung: **07.11.2007**
(21) Anmeldenummer: 03704196.9
(22) Anmeldetag: 07.01.2003
(51) Int. Cl.: A61L 27/28, A61L 27/34, A61L 31/10, C07K 14/51, G06F 17/50, C07K 1/00

(54) **VERFAHREN ZUR SPEZIFISCHEN IMMOBILISIERUNG VON MEDIATORMOLEKUELEN AUF SUBSTRATOBERFLAECHEN**
METHOD FOR SPECIFICALLY IMMOBILIZING MEDIATOR MOLECULES ON SUBSTRATE SURFACES
PROCEDE D'IMMOBILISATION SPECIFIQUE DE MOLECULES MEDIATEURS SUR DES SURFACES DE SUBSTRAT

(30) Priorität: 04.01.2002 DE 10200161
(43) Veröffentlichungstag der Anmeldung: 06.10.2004
(73) Patentinhaber: Jennissen, Herbert P., 50858 Köln (DE); Laub, Markus, 45134 Essen (DE)
(72) Erfinder: Jennissen, Herbert P., 50858 Köln (DE); Laub, Markus, 45134 Essen (DE)
(74) Vertreter: Viering, Jentschura & Partner
(86) Internationale Anmeldenummer: PCT/DE2003/000033
(87) Internationale Veröffentlichungsnummer: WO 2003/059954

(56) Entgegenhaltungen:
- WO-A-00/52457
- WO-A-02/18952
- WO-A-99/26674
- M LAUB ET AL.: "Molecular modellin of bone morphogenetic protein-2 (BMP-2) by 3-D rapid prototyping" MATERIALWISSENSCHAFT UND WERKSTOFFTECHNIK., Bd. 32, 2001, Seiten 926-930, XP008019536 VCH VERLAGSGESELLSCHAFT, WEINHEIM., DE ISSN: 0933-5137
- H P JENNISSEN ET AL.: "Biocoating of implants with mediator molecules : surface enhancement of metals by treatment with chromosulphuric acid" MATERIALWISSENSCHAFT UND WERKSTOFFTECHNIK., Bd. 30, 1999, Seiten 838-845, XP008019584 VCH VERLAGSGESELLSCHAFT, WEINHEIM., DE ISSN: 0933-5137
- G VOGGENREITER ET AL.: "Biologische Beschichtung von Implantaten mit rhBMP-2" CHIRURGISCHES FORUM FUER EXPERIMENTELLE UND KLINISCHE FORSCHUNG, Bd. 30, 2001, Seiten 452-454, XP008019579 BERLIN, DE ISSN: 0303-6227

## Beschreibung

Die Erfindung betrifft ein Verfahren zur spezifischen Immobilisierung von Mediatormolekülen auf Substratoberflächen bei dem in einem ersten Schritt Ankermoleküle mit einer Helix-Struktur an die chemisch aktivierte Oberfläche des Substrates kovalent gebunden werden, und in einem zweiten Schritt Mediatormoleküle mit einer Anthelix-Struktur auf der Substratoberfläche immobilisiert werden, wobei als Mediatormoleküle Wachstumsfaktoren aus der Klasse der TGF-beta-Faktoren, insbesondere BMP-Proteine verwendet werden.

Knochenmorphogenetische Proteine (BMPs) spielen eine entscheidende Rolle in der Entwicklung des Knochens und der Osteogenese. In der Vergangenheit waren sie als Stimulanzien des Knochenwachstums Gegenstand zahlreicher Forschungsvorhaben und klinischer Studien. Kürzlich wurde BMP-2 chemisch auf Implantat-Oberflächen immobilisiert, was bei Schafen zu verstärktem Knochenwachstum und beschleunigter Integration führte. Obwohl die 3D-Struktur von BMP-2 bekannt ist, war die Topographie seiner Oberfläche bisher nicht Thema einer detaillierten Analyse.

Seitens der Erfinder wurde gefunden, dass die Technik des 3D-Rapid Protoyping als neue Methode zur Gewinnung topographischer Informationen über die Struktur-Funktions-Beziehung von Proteinen eingesetzt werden kann. Das 3D-Rapid Prototyping erlaubt die Konstruktion exakter dreidimensionaler Modelle von Proteinen, basierend auf deren Röntgenstrukturdaten oder NMR-Daten.

Der Prozess vom Design über den Prototypen bis hin zur Serie kann grundsätzlich mit Hilfe von Rapid Prototyping flexibel, zeit- und kostensparend gestaltet werden. Im Stand der Technik bekannte, so hergestellte Prototypen helfen, das Produktdesign zu verbessern, Entwicklungszyklen zu reduzieren und die Produktqualität zu erhöhen.

Das Rapid-Prototyping-Verfahren, insbesondere das Verfahren des selektiven Lasersinterns (SLS), der Stereolithographie, des Fused Deposition Modeling (FDM) oder des Laminated Object Manufacturing bietet die Möglichkeit, auch sehr komplexe Geometrien z.B. mit Hohlräumen und Hinterschnitten zu realisieren, die mit anderen Verfahren nur sehr schwer oder sogar überhaupt nicht erstellt werden können. Für das selektive Lasersintern können die Geräte der SLS-Serie der Firma EOS (München), für das Stereolithographie-Verfahren beispielsweise die Geräte der SLA-Series der Fa. 3D-Systems eingesetzt werden.

In dem Rapid-Prototyping-Verfahren wird zunächst das anhand von 3D-Strukturdaten, bevorzugt Röntgenstrukturdaten und/oder NMR-Daten, vorhandene 3D-Modell mit Hilfe der STL-Schnittstelle (Standard Transformation Language) in einen standardisierten Datensatz transformiert. Der STL-Datensatz beschreibt die Molekülstruktur, -geometrie und Oberfläche durch kleinste Dreiecke (Triangulierung). Aus diesem Datensatz werden auf einem Prozeßrechner mit vom RP-Verfahren abhängiger Software Querschnitte in der X-Y-Ebene (Slicen) generiert und auf die Sinterungsmaschine übertragen.

Beim Lasersinterungsprozeß wird so beispielsweise ein Polyamidpulver mit einem Infrarot-Laserstrahl gemäss dem STL-Datensatz zunächst selektiv geschmolzen und dann verfestigt (gesintert). Dann wird die Molekülplattform abgesenkt und eine neue dünne Schicht Polyamidpulver aufgetragen. Durch Wiederholung dieser Schritte wird sukzessive die vollständige Geometrie von unten nach oben aufgebaut. Das so entstandene Molekül kann abschließend nachgehärtet und entgratet werden.

Die so hergestellten SLS-Moleküle zeichnen sich durch eine sehr hohe Maß- und Formhaltigkeit aus und können je nach Anwendung auch als Funktionsmodelle eingesetzt werden.

Auf diese Weise konnten die Erfinder ein maßstabsgetreues 3D-Abbild von BMP-2 mit den Maßen 140 x 70 x 50 mm, was einer Vergrößerung von 20 x 106 entspricht (Maßstab 1 nm = 2 cm), herstellen. BMP-2 stellt sich als gedrehtes bananen-förmiges Molekül mit einer konvexen und einer konkaven Seite dar und besitzt an den Enden zwei hornähnliche Vorsprünge, die gegeneinander um 180 C (Längsachse) verdreht sind. Im Zentrum der konvexen Seite befindet sich eine ca. 1 nm tiefe kraterähnliche Vertiefung mit einem Durchmesser von ca. 1.8 nm. Die konkave Seite ist durch einen 6-7 nm lange, 0.8-1.6 nm breite und ca. 0.8 nm tiefe helikale Furche gekennzeichnet, in die eine linksgängige Helix mit einer Ganghöhe von 8-9 nm und einem helikalen Radius von 0.35 0.45 nm eingepasst werden kann. Die konkave Seite des BMP-2 entspricht daher dem Abdruck (Furche) einer linksgängigen Helix d.h. einer Anti-Helix oder Anthelix. Mögliche endogene Liganden und Funktionen dieser Struktur sind unbekannt. Diese Ergebnisse zeigen, daß 3D-Modelle von Proteinen durch makroskopisches Betrachten und Anpassen von Molekülen mit der Hand ohne Zuhilfenahme eines Computers zu neuen Erkenntnissen über die Interaktion zwischen Liganden und Proteinen führen können.

Für das BMP-2 wurde seitens der Erfinder festgestellt, dass es sich dabei um ein Anthelix-Protein handelt. Weitere Anthelix-Proteine sind TGF-β, BMP-7 und andere BMPs. Diese Anthelix-Struktur dieser Proteine ist bisher unbekannt gewesen.

Die Proteine der TGF-beta-(Transforming Growth Factor beta)-Familie (Molekulargewicht in der Regel 20-30 kDa) zeichnen sich in der Regel durch eine homodimere Struktur aus, die im Bereich der intermolekularen Disulfidbrücke einen Cystin-Knoten besitzt und auf der konkaven Seite durch eine Anthelix ausgezeichnet ist.

Bei diesen Wachstumsfaktoren der TGF-beta-Klasse handelt es sich um extrazelluläre multifunktionale Signalproteine, die an heterodimere Rezeptoren (Serin-Proteinkinasen) binden sowie Wachstum und Morphogenese im Vielzeller steuern.

Die wichtigste Untergruppe der TGF-beta-Familie stellen die "Bone morphogenetic proteins" (BMPs) dar. In Säugern wurden bisher mindestens 15 verschiedene BMPs beschrieben. Diese Proteine steuern z.B. während der Entwicklung eines Embryos die Bildung von Knochen, Zähnen und Knorpeln. Im erwachsenen Säuger steuert das BMP-2 vor allem Knochenwachstum- und Knochenheilung.

Die Erfinder haben festgestellt, dass BMP-2 auf der Unterseite eine Anthelix (= Minus-Helix, Negativ) besitzt, in die eine Plus-Helix (Positiv) mit folgenden Charakteristika hineinpaßt: linkshändig, Ganghöhe 8-9 nm, helikaler Radius 0.35-0,45 nm. Theoretisch könnte ein Protein, Nukleinsäure, Kohlenhydrat oder Lipid eine solche Helix bilden. Die physiologische Plus-Helix für die BMP-2 Anthelix ist nicht bekannt. Aber es ist den Erfindern gelungen, zwei Plus-Peptide 1 und 2 mit den unten angegebenen Sequenzen aufzufinden, die in die Anthelix des BMP-2 hineinpassen. Das Peptid 1 ist im Stand der Technik bekannt und hat folgende Sequenz: LYS-ASN-MET-THR-PRO-TYR-ARG-SER-PRO-PRO-PRO-TYR-VAL-PRO-PRO. Das o.g. Peptid besteht aus 15 L-Aminosäuren. Solche Peptide können von Proteasen abgebaut werden.

So ist es aber auch möglich, eine Plus-Helix aus D-Aminosäuren zu synthetisieren, die proteolysestabil ist. Daher werden erfindungsgemäß auch D-Aminosäure-Peptide berücksichtigt.

Ein so hergestelltes Protein kann bei der Herstellung von Wachstumsfaktoren mittels Affinitätschromatographie zur Reinigung von Anthelix-Proteinen der TGF-Klasse, insbesondere der BMPs, hiervon insbesondere von BMP-2, in einem Schritt verwendet werden. Es kann eine chemische Kopplung des Peptids z.B. am Lysin auf Gelen für affinitätschromatographische Verfahren an Kohlenhydratgele (Agarose, Cellulosegele, etc.), Kieselgele, poröses Glas, Metallpulver erfolgen. Die Erfindung betrifft somit auch Chromatographieverfahren, bei denen stationäre Phasen verwendet werden, an die Ankermoleküle mit einer Helixstruktur gebunden sind, sowie die in diesen Chromatographieverfahren verwendeten stationären Phasen. Die Helixstruktur der Ankermoleküle ist zumindest für den Teilbereich des Ankermoleküls, der mit dem Wachstumsfaktor, der auch in dimerer Form vorliegen kann, in Wechselwirkung treten soll, notwendig.

Die chemischen Kopplungsverfahren für das Plus-Peptid sind im Stand der Technik bekannt. Bei den Substraten ist vorzugsweise eine aktivierende Behandlung der Substratoberfläche mittels ebenfalls im Stand der Technik bekannter Aktivierungsverfahren möglich. BMP-2 kann dann an dem vorzugsweise nach Aktivierung auf der Substratoberfläche immobilisierten Peptid hochspezifisch adsorbiert und dann mit geeigneten Verfahren (löslichem Peptid, SDS, Harnstoff, Guanidinium-Hydrochlorid) eluiert werden.

Mittels des erfindungsgemäßen Verfahrens kann auf diese Weise auch die Biologisierung von Implantaten mit Anthelix-Proteinen (BMP-2, BMP-7, TGF-β-Proteine) erfolgen.

So kann BMP-2 auf metallischen Implantaten zur Anregung des Knochenwachstums durch hochspezifische Adsorption an das Plus-Peptid (indirekt) immobilisiert werden. Die chemischen Kopplungsverfahren für das Plus-Peptid sind grundsätzlich im Stand der Technik bekannt. Eine Vorbehandlung der metallischen Oberfläche mit Chromschwefelsäure kann nach Bedarf erfolgen.

Ein weiterer Vorteil des erfindungsgemäßen Verfahrens neben der hochspezifischen Bindung ist, dass beim Wegdiffundieren von BMP-2 von der Plus-Helix auf der Implantatoberfläche dort wieder hochspezifisch neues körpereigenes BMP-2 gebunden werden kann. Es handelt sich also um eine Art "wiederbeladbare Implantatoberfläche".

### Materialien und Methoden

### Prinzipien für die Bindung von Liganden an die Anti-Helix (Anthelix) von TGF-beta Proteinen

### 1. Plus-Helix Eigenschaften

Die Erfinder haben herausgefunden, dass der Ligand (z.B. Polypeptid) komplementär zur Anti-Helix (Minus-Helix) im Dimer sein und dort binden muss. Die notwendige Wechselwirkungsenergie lässt sich mittels des Docking-Programmes GRAMM 1.03 bestimmen.

### 2. Mindestenergie nach Docking-Programm GRAMM 1.03

Um im erfindungsgemäßen Sinne verwendbar zu sein, muss das Ankermolekül eine hohe Bindungsaffinität besitzen, die über den auf Energieeinheiten beruhenden Energiequotienten (Q) abgeschätzt werden kann (siehe Tabelle 1 und Peptid-Sequenzen 1 und 2 unten). Der Energiequotient wird berechnet zu Q = arbiträre Energieeinheiten (Protein)/ arbiträre Energieeinheiten (TGF-beta-Dimer) und sollte erfindungsgemäß bevorzugt einen Wert von >0.4, bevorzugt >0.5 und besonders bevorzugt >0.65 im nicht optimierten Berechnungssystem, dem "Low-resolution generic docking"-Modus erreichen. Der Energiequotient wird mit Hilfe des Docking-Programmes GRAMM 1.03 (http://reco3.ams.sunysb.edu/gramm; Vakser, I.A., Medical University of South Carolina) ermittelt, das nachfolgend beschrieben wird.

Die Erfindung betrifft daher insbesondere ein Verfahren zur spezifischen Immobilisierung von Mediatormolekülen, bei dem die Ankermoleküle, bevorzugt Peptide mit einer Anzahl Aminosäuren von etwa 5 bis 40, mehr bevorzugt 10 bis 35, mit einer Helix-Struktur in Abhängigkeit von den zu immobilisierenden Mediatormolekülen mit Anthelix-Struktur so ausgewählt werden, dass der Energiequotient Q = arbiträre Energieeinheiten (Ankermolekül) / arbiträre Energieeinheiten (TGF-beta-Dimer) bevorzugt einen Wert von >0.4, mehr bevorzugt >0.5 und besonders bevorzugt >0.65 im nicht optimierten Berechnungssystem gemäß dem "Low-resolution generic docking"-Modus, berechnet nach dem Docking-Programmes GRAMM 1.03, besitzt.

Bevorzugt sollte das Protein mit der Plus-Helix-Struktur, die zumindest in einem Teilbereich vorhanden ist und mit dem TGF-beta-Protein in Wechselwirkung tritt, an diesen TGF-beta-Faktor in seiner dimeren Form, und weniger stark an das TGF-beta-Proteine in seiner monomeren Form binden. Das Verhältnis der arbiträre Energieeinheiten (Protein) / arbiträre Energieeinheiten (TGF-beta-Dimer) zu arbiträre Energieeinheiten (Protein)/ arbiträre Energieeinheiten (TGF-beta-Monomer) sollte erfindungsgemäß bevorzugt einen Wert von mehr als 1,15, mehr bevorzugt von mehr als 1,3 beim nicht optimierten Berechnungssystem, dem "Low-resolution generic docking"-Modus erreichen.

Die Erfindung betrifft daher insbesondere auch ein Verfahren zur spezifischen Immobilisierung von Mediatormolekülen, bei dem die Ankermoleküle mit einer Helix-Struktur in Abhängigkeit von den zu immobilisierenden Mediatormolekülen mit der Anthelix-Struktur so ausgewählt werden, dass das Verhältnis der arbiträren Energieeinheiten (Anklermolekül) / arbiträre Energieeinheiten (TGF-beta-Dimer) zu arbiträre Energieeinheiten (Protein) / arbiträre Energieeinheiten (TGF-beta-Monomer) einen Wert von mehr als 1,15, bevorzugt von mehr als 1,3 beim nicht optimierten Berechnungssystem gemäß dem "Low-resolution generic docking"-Modus, berechnet nach dem Docking-Programmes GRAMM 1.03, besitzt.

Dieses Docking-Programm wurde ausführlich an Modellsystemen überprüft, die in den Referenzen 1-3 unten angegeben sind. Das Programm benutzt für die Suche nach möglichen Bindungsstellen einen geometrischen Erkennungsalgorithmus. Als Inputdateien für GRAMM dienen Röntgen- bzw. NMR-Strukturdaten aus der Brookhaven-Datenbank (http://www.rcsb.org), die die 3D-Struktur der Moleküle definieren. Die Steuerung des Programms erfolgt über ASCII-Dateien (Rpar.gr, Rmol.gr, Wlist.gr), die verschiedene Parameter enthalten. Hier kann u.a. durch die Wahl der Auflösung (Rpar.gr) der "Low-resolution generic docking"-Modus eingestellt werden, der erfindungsgemäß eingesetzt wird. Als Ergebnis liefert GRAMM eine Liste möglicher Anordnungen der miteinander in Wechselwirkung gesetzten beiden Moleküle, die nach arbiträren Energieeinheiten ("energy values") für die einzelnen Anordnungen größenmäßig sortiert ist. Im Sinne der Erfindung wird die Anordnung der miteinander in Wechselwirkung gesetzten beiden Moleküle des angepassten Modells ausgewählt, die den höchsten arbiträren Energieeinheiten entspricht. Zusätzlich muss diese Anordnung die Bindungsstelle in der Anthelixregion aufweisen (siehe oben).
[1] Katchalski-Katzir, E., Shariv, I., Eisenstein, M., Friesem, A.A., Aflalo, C., & Vakser, I.A., Molecular surface recognition: determination of geometric fit between proteins and their ligands by correlation techniques. Proc. Natl. Acad. Sci. U. S. A 89 (1992), 2195-2199.
[2] Vakser, I.A., Protein docking for low-resolution structures. Protein Eng. 8 (1995), 371-377.
[3] Vakser, I.A., Low-resolution docking: prediction of complexes for underdetermined structures. Biopolymers 39 (1996), 455-464.

**Tabelle 1**

| Molekulare Dockinganalyse von Peptidwechselwirkungen mit BMP-2 | | | | |
|---|---|---|---|---|
| Bindungspeptid | | Bindungspartner | Anpassungsgüte | Energie-quotient (Q) |
| Name | Länge (Anzahl der Aminosäuren) | | arbiträre Energieeinheiten | |
| Kontrolle BMP-2 Monomer | 114 | BMP-2 Monomer | 329 | 1.0 |
| Plus-Peptid 1 Lys1-Pro14 | 14 | BMP-2 Dimer | 158 | 0.48 |
| Plus-Peptid 1 Lys1-Pro14 | 14 | BMP-2 Monomer | 134 | 0.41 |
| Plus-Peptid 2 Cys1-Glu34 | 34 | BMP-2 Dimer | 234 | 0.71 |
| Plus-Peptid 2 Cys1-Glu34 | 34 | BMP-2 Monomer | 176 | 0.54 |

| | | | | |
|---|---|---|---|---|
| Plus-Peptid 1 (Lys1-Pro14) LYS-ASN-MET-THR-PRO-TYR-ARG-SER-PRO-PRO-PRO-TYR-VAL-PRO-PRO | | | | |

Zur Herstellung eines Modells des Knochenwachstumsfaktors BMP-2 wurde die Quartärstruktur des dimeren BMP-2 aus der EMBL-Datenbank ("Protein Quaternary Structure File Server" heruntergeladen. Der verwendete Rechenalgorithmus berechnet die Koordinaten für die wahrscheinlichsten quarternären Zustände der Moleküle in der Brookhaven Protein Data Bank. Das dimere BMP-2 wurde aus den Röntgenstrukturdaten für das monomere BMP-2 berechnet.

Die "solvent-accessible surface" (flüssigkeitszugängliche Oberfläche) des dimeren BMP-2 wurde mit Hilfe des "molecular graphics" Programmes VMD 1.61 mit Hilfe des SURF Moduls von A. Varshney berechnet. Die "solvent-accessible surface" ist definiert als die Oberfläche, die von einer darüberliegenden "Probekugel" berührt werden kann, wenn diese über die kugelförmigen Atome gerollt wird. Die dadurch resultierende glatte "Monoschichtoberfläche" besteht aus den Anteilen der van der Waals Oberflächen der Atome, die der Probekugel zugänglich waren. Der verwendete Probenkugelradius betrug 1.4 Angström, welches dem Radius eines kugelförmig approximierten Wassermoleküls entspricht. Die Datenfiles mit den Koordinaten für die "solvent-accessible surface" wurden in das STL-Format mit dem Programm VMD 1.61 übersetzt.

Die 3D-Modelle des BMP-2 wurden mit dem Lasersinterungsgerät EOSINT P360 (EOS GmbH, Planegg/München) hergestellt. Der Schichtherstellungsprozeß findet in einer Prozeßkammer mit inerter Gasatmosphäre statt. Pulverförmiges Plastikmaterial wird in dünnen Schichten auf eine beheizte Konstruktionsplattform aufgetragen, deren Temperatur gerade unterhalb des Schmelzpunktes des Plastikpulvers liegt. Ein computergesteuerter Infrarot-Laser bewirkt nun eine lokale Erhitzung des Pulvers über den Schmelzpunkt mit anschließender Aushärtung (Sinterung). Das BMP-2 Modell wurde in einer Größe von ca. 140 x 70 x 50 mm hergestellt.

Die spiralförmige Struktur auf der konkaven Seite des BMP-2 wurde mit einem Moosgummischlauch (Länge 35 cm, Außendurchmesser 10 mm, Innendurchmesser 2 mm), der eine Drahtseele enthielt, sondiert und angepasst.

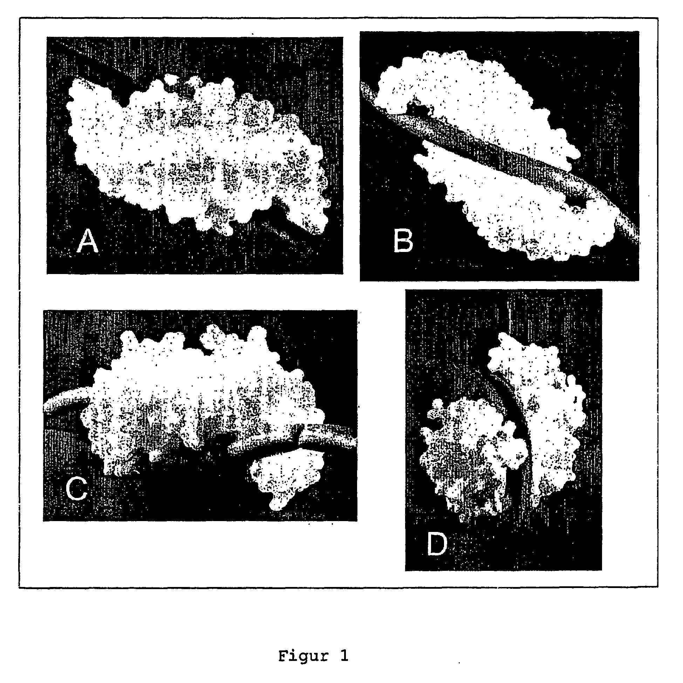

Die Erfindung wird anhand der Figur 1 weiter erläutert.

### Beispiel

Die Oberflächentopographie des BMP-2 und insbes. die helikale Furche auf der Unterseite, wie man sie in Fig. 1 sieht, ist in einem 3D-Modell des Moleküls auf einem Computerbildschirm nur schwer erkennbar und wurde daher bis jetzt noch nicht beschrieben. Ein Grund für diesen Mangel ist, dass auch ein 3D-Modell auf einem Computerbildschirm nur 2-dimensional dargestellt werden kann, auch wenn das Modell gedreht oder manipuliert wird. Alle Oberflächenstrukturen werden in einer Ebene auf dem Bildschirm projiziert, so dass Vertiefungen wie Furchen oder Gruben unerkannt bleiben.

Wie Fig. 1 zeigt, kann eine linksspiralige Furche (Anthelix, Negativhelix) mit einer Ganghöhe von 8-9 nm und einem Radius von ca. 0.35-0.40 nm mit einer nachgearbeiteten Modellsonde als "Plushelix" eindeutig nachgewiesen werden. Die natürliche "Plushelix" für BMP-2 ist jedoch unbekannt. Die anfängliche Annahme der Erfinder, daß eine Kollagen-Tripelhelix (Ganghöhe 8.6 nm, 0.5-7 nm) gut in diese Furche passen würde, wurde durch die Untersuchungen der Erfinder widerlegt, da die Kollagen-Tripelhelix nicht passt, da es sich um eine rechtshändige Helix handelt. Dies konnten die Erfinder auch mit dem "molecular-docking" Programm per Computersimulation zeigen. Auch andere bekannte Helices wie die Alpha-Helix oder die pi-Helix mit einer Ganghöhe von 5.4-6 nm passen nach Untersuchungen der Erfinder nicht. Lediglich das oben angegebene 15er-Peptid 1 und das 34er Peptid 2 lassen sich in einem "molecular-docking"-Programm per Computer gut in die Furche des BMP-2 einpassen.

### Figurenlegende

Wie in Figur 1 gezeigt, ist dort eine helikale Furche (Anthelix) auf der konkaven Seite von BMP-2 mittels eines eingepaßten Spiralschlauches zu erkennen.

Die spiralige Struktur im BMP-2 (Anthelix) wurde mit einem linkshelikal geformten Moosgummischlauch (Sonde) von 1 cm Durchmesser (~0.5 nm) zu einer Helixganghöhe von 16-18 cm (ca. 8-9 nm) und einem Helixradius von 0.6-0.7 cm (0.35-0.4 nm) sondiert und eingepaßt. Dabei zeigen in Figur 1:
A. eine Ansicht der konvexen Seite von BMP-2 das sattelförmig auf der Moosgummisonde liegt;
B. eine Ansicht der konkaven Seite mit der eingepaßten Moosgummisonde, die in der Anthelix festgehalten wird;
C. eine Seitenansicht von BMP-2 mit der eingepaßten Moosgummisonde in der Anthelix unterhalb des BMP-2 Moleküls; sowie
D. eine Ansicht vom Ende her entlang der Helixachse mit eindeutiger Darstellung der eingepaßten links-helikalen Moosgummisonde in der Anthelix des BMP-2.

## Patentansprüche

1. Verfahren zur spezifischen Immobilisierung von Mediatormolekülen auf Substratoberflächen, **dadurch gekennzeichnet, dass** in einem ersten Schritt Ankermoleküle mit einer Helix-Struktur an die bevorzugt chemisch aktivierte Oberfläche des Substrates kovalent gebunden werden, und in einem zweiten Schritt Mediatormoleküle mit einer Anthelix-Struktur auf der Substratoberfläche infolge Helix-Anthelix-Wechselwirkungen mit den Ankermolekülen immobilisiert werden,
wobei als Mediatormoleküle Wachstumsfaktoren aus der Klasse der TGF-beta-Proteine, insbesondere der BMP-Proteine, verwendet werden.

2. Verfahren nach Anspruch 1, wobei als Wachstumsfaktoren die Knochenwachstumsfaktoren BMP-2 oder BMP-7 einzeln oder im Gemisch verwendet werden.

3. Verfahren nach Anspruch 2, wobei als Knochenwachstumsfaktor BMP-2 und als Ankermolekül ein Peptid, das bevorzugt die Sequenz LYS-ASN-MET-THR-PRO-TYR-ARG-SER-PRO-PRO-PRO-TYR-VAL-PRO-PRO oder CYS-ALA-VAL-GLU-LEU-ARG-SER-PRO-GLY-ILE-SER-ARG-PHE-ARG-ARG-LYS-ILE-ALA-LYS-ARG-SER-ILE-LYS-THR-LEU-GLU-HIS-LYS-ARG-GLU-ASN-ALA-LYS-GLU umfasst, verwendet wird.

4. Verfahren nach einem der Ansprüche 1 bis 3, bei dem die Ankermoleküle mit einer Helix-Struktur in Abhängigkeit von den zu immobilisierenden Mediatormolekülen mit Anthelix-Struktur so ausgewählt werden, dass der Energiequotient Q = arbiträre Energieeinheiten (Ankermolekül) / arbiträre Energieeinheiten (TGF-beta-Dimer) bevorzugt einen Wert von >0.4, mehr bevorzugt >0.5 und besonders bevorzugt >0.65 im nicht optimierten Berechnungssystem gemäß dem "Low-resolution generic docking"-Modus, berechnet nach dem Docking-Programmes GRAMM 1.03, besitzt.

5. Verfahren nach einem der Ansprüche 1 bis 4, bei dem die Ankermoleküle mit einer Helix-Struktur in Abhängigkeit von den zu immobilisierenden Mediatormolekülen mit der Anthelix-Struktur so ausgewählt werden, dass das Verhältnis der arbiträren Energieeinheiten (Anklermolekül) / arbiträre Energieeinheiten (TGF-beta-Dimer) zu arbiträre Energieeinheiten (Protein) / arbiträre Energieeinheiten (TGF-beta-Monomer) einen Wert von mehr als 1,15, bevorzugt von mehr als 1,3 beim nicht optimierten Berechnungssystem gemäß dem "Low-resolution generic docking"-Modus, berechnet nach dem Docking-Programmes GRAMM 1.03, besitzt.

6. Verfahren nach einem der Ansprüche 1 bis 5, bei dem als Substrat die stationäre Phase eines chromatographischen Trennsystems eingesetzt wird.

7. Verfahren nach einem der Ansprüche 1 bis 5, bei dem als Substrat ein Implantatmaterial eingesetzt wird, das aus einem Material aus der Gruppe der Metalle, der metallischen Legierungen, der keramischen Materialien oder Kombinationen davon ausgewählt wird.

8. Verfahren nach Anspruch 7, **dadurch gekennzeichnet, dass** die Oberfläche des Implantatmaterials vor der kovalenten Bindung der Ankermoleküle mit einer Oxidschicht versehen wird.

9. Verfahren nach Anspruch 8, **dadurch gekennzeichnet, dass** die Oberfläche des Implantatmaterials, ausgewählt aus Titan, Titanlegierungen, Aluminium oder rostfreiem Stahl, vor der kovalenten Bindung der Ankermoleküle durch Behandlung mit Chromschwefelsäure über einen Zeitraum von 0,5 bis zu 3 Stunden bei 100 bis 250°C mit einer Oxidschicht versehen wird.

10. Verfahren nach Anspruch 9, **dadurch gekennzeichnet, dass** die Chromschwefelsäure eine Dichte von mehr 1,40 g/cm³ hat.

11. Modifiziertes Substrat, erhältlich nach dem Verfahren nach einem der vorhergehenden Ansprüche.

12. Verwendung des modifizierten Substrats nach Anspruch 11, als stationäre Phase oder eines Teils der stationären Phase eines chromatographischen Trennsystems, insbesondere zur Aufreinigung von Wachstumsfaktoren.

## Claims

1. Process for specifically immobilising mediator molecules on substrate surfaces, **characterised in that**, in a first step, anchor molecules having a helix structure are covalently bonded to the substrate surface, which is preferably chemically activated, and, in a second step, mediator molecules having an anthelix structure are immobilised on the substrate surface as a result of helix-anthelix interactions with the anchor molecules,
growth factors from the class of the TGF beta proteins, especially the BMP proteins, being used as the mediator molecules.

2. Process according to claim 1, wherein the bone growth factors BMP-2 or BMP-7 are used individually or in admixture as the growth factors.

3. Process according to claim 2, wherein BMP-2 is used as the bone growth factor and a peptide which preferably includes the sequence LYS-ASN-MET-THR-PRO-TYR-ARG-SER-PRO-PRO-PRO-TYR-VAL-PRO-PRO or CYS-ALA-VAL-GLU-LEU-ARG-SER-PRO-GLY-ILE-SER-ARG-PHE-ARG-ARG-LYS-ILE-ALA-LYS-ARG-SER-ILE-LYS-THR-LEU-GLU-HIS-LYS-ARG-GLU-ASN-ALA-LYS-GLU is used as the anchor molecule.

4. Process according to any one of claims 1 to 3, wherein the anchor molecules having a helix structure are selected in dependence on the mediator molecules to be immobilised having an anthelix structure in such a manner that the energy quotient Q = arbitrary energy units (anchor molecule)/arbitrary energy units (TGF beta dimer) preferably has a value of >0.4, more preferably >0.5 and especially preferably >0.65 in the non-optimised calculation system in accordance with the low-resolution generic docking mode, calculated according to the docking program GRAMM 1.03.

5. Process according to any one of claims 1 to 4, wherein the anchor molecules having a helix structure are selected in dependence on the mediator molecules to be immobilised having the anthelix structure in such a manner that the ratio of the arbitrary energy units (anchor molecule)/arbitrary energy units (TGF beta dimer) to arbitrary energy units (protein)/arbitrary energy units (TGF beta monomer) has a value of more than 1.15, preferably more than 1.3 in the case of the non-optimised calculation system in accordance with the low-resolution generic docking mode, calculated according to the docking program GRAMM 1.03.

6. Process according to any one of claims 1 to 5, wherein the stationary phase of a chromatographic separation system is used as the substrate.

7. Process according to any one of claims 1 to 5, wherein an implant material selected from a material from the group consisting of metals, metal alloys, ceramic materials or combinations thereof is used as the substrate.

8. Process according to claim 7, **characterised in that** the surface of the implant material is provided with an oxide layer before the covalent bonding of the anchor molecules.

9. Process according to claim 8, **characterised in that** the surface of the implant material, selected from titanium, titanium alloys, aluminium or stainless steel, is provided with an oxide layer by treatment with chromosulphuric acid over a period of from 0.5 to 3 hours at from 100 to 250°C before the covalent bonding of the anchor molecules.

10. Process according to claim 9, **characterised in that** the chromosulphuric acid has a density of more than 1.40 g/cm³.

11. Modified substrate, obtainable in accordance with the process according to any one of the preceding claims.

12. Use of the modified substrate according to claim 11, as the stationary phase or part of the stationary phase of a chromatographic separation system, especially for purifying growth factors.

## Revendications

1. Procédé pour l'immobilisation spécifique de molécules de médiateurs sur des surfaces de substrat, **caractérisé en ce que**, dans une première étape des molécules d'ancrage ayant une structure en hélice sont fixées par liaison covalente à la surface de préférence chimiquement activée du support, et, dans une deuxième étape des molécules de médiateurs ayant une structure en anti-hélice sont immobilisées sur la surface du support, par suite d'interactions hélice/anti-hélice avec les molécules d'ancrage,
où on utilise en tant que molécules de médiateurs, des facteurs de croissance choisis dans la classe des protéines-TGF-bêta, notamment des protéines-BMP.

2. Procédé selon la revendication 1, dans lequel on utilise comme facteurs de croissance les facteurs de croissance osseux BMP-2 ou BMP-7, individuellement ou en mélange.

3. Procédé selon la revendication 2, dans lequel on utilise en tant que facteur de croissance de osseux BMP-2 et en tant que molécule d'ancrage un peptide qui a de préférence la séquence LYS-ASN-MET-THR-PRO-TYR-ARG-SER-PRO-PRO-PRO-TYR-VAL-PRO-PRO ou CYS-ALA-VAL-GLU-LEU-ARG-SER-PLO-GLY-ILE-SER-ARG-PHE-ARG-ARG-LYS-ILE-ALA-LYS-ARG-SER-ILE-LYS-THR-LEU-GLU-HIS-LYS-ARG-GLU-ASN-ALA-LYS-GLU.

4. Procédé selon l'une quelconque des revendications 1 à 3, dans lequel les molécules d'ancrage ayant une structure en hélice sont choisies en fonction des molécules de médiateurs à structure en anti-hélice, à immobiliser, de manière que le quotient d'énergie Q = unités d'énergie arbitraires (molécule d'ancrage) / unités d'énergie arbitraires (dimère de TGF-bêta) ait de préférence une valeur > 0,4, plus préférentiellement >0,5 et de façon particulièrement préférée >0,65 dans le système de calcul non optimisé selon le mode "Low resolution generic docking", calculée selon le programme Docking GRAMM 1.03.

5. Procédé selon l'une quelconque des revendications 1 à 4, dans lequel les molécules d'ancrage ayant une structure en hélice sont choisies en fonction des molécules de médiateurs ayant la structure en hélice opposée, à immobiliser, de manière que le rapport des unités d'énergie arbitraires (molécule d'ancrage) / unités d'énergie arbitraires (dimère de TGF-bêta) aux unités d'énergie arbitraires (protéine) / unités d'énergie arbitraires (monomère de TGF-bêta) ait une valeur de plus de 1,15, de préférence de plus de 1,3 dans le système de calcul non optimisé selon le mode "Low resolution generic docking", calculée selon le programme Docking GRAMM 1.03.

6. Procédé selon l'une quelconque des revendications 1 à 5, dans lequel on utilise comme substrat la phase stationnaire d'un système de séparation chromatographique.

7. Procédé selon l'une quelconque des revendications 1 à 5, dans lequel on utilise comme substrat un matériau pour implant qui est choisi parmi un matériau appartenant au groupe des métaux, les alliages métalliques, des matériaux céramiques, ou des associations de ceux-ci.

8. Procédé selon la revendication 7, **caractérisé en ce que** la surface du matériau pour implant est munie d'une couche d'oxyde avant la fixation par liaison covalente des molécules d'ancrage.

9. Procédé selon la revendication 8, **caractérisé en ce que** la surface du matériau pour implant, choisi parmi le titane, des alliages de titane, l'aluminium ou l'acier inoxydable, est munie d'une couche d'oxyde, avant la fixation par liaison covalente des molécules d'ancrage, issue d'un traitement par de l'acide sulfochromique pendant une durée de 0,5 à 3 heures à 100-250 °C.

10. Procédé selon la revendication 9, **caractérisé en ce que** l'acide sulfochromique a une densité de plus de 1,40 g/cm³.

11. Substrat modifié, pouvant être obtenu conformément au procédé selon l'une quelconque des revendications précédentes.

12. Utilisation du substrat modifié selon la revendication 11, en tant que phase stationnaire ou une partie de la phase stationnaire d'un système de séparation chromatographique, en particulier pour la purification de facteurs de croissance.


### SEQUENZ-LISTE

<110> Prof. Dr. H. P. Jennissen
   Institut für Physiologische Chemie
   Universitätsklinikum Essen
   Hufelandstraße 55
   45122 Essen
   Deutschland
<120> Verfahren zur spezifischen Immobilisierung von Biomolekülen
<130> P 200347
<140>
   <141>
<160> 2
<170> PatentIn Version 3.0
<210> 1
   <211> 15
   <212> PRT
   <213> Künstliche Sequenz
<400> 1
   Lys Asn Met Thr Pro Tyr Arg Ser Pro Pro Pro Tyr Val Pro Pro
   1               5                   10                  15
<210> 2
   <211> 34
   <212> PRT
   <213> Künstliche Sequenz
<400> 2
   CYS ALA VAL GLU LEU ARG SER PRO GLY ILE SER ARG PHE ARG ARG
   1               5                   10                  15
   LYS ILE ALA LYS ARG SER ILE LYS THR LEU GLU HIS LYS ARG GLU
                   20                  25                  30
   ASN ALA LYS GLU
               34